# EUROPÄISCHE PATENTSCHRIFT

(11) **EP 2 189 254 B1**
(45) Veröffentlichungstag und Bekanntmachung des Hinweises auf die Patenterteilung: **03.07.2013**
(21) Anmeldenummer: 08169866.4
(22) Anmeldetag: 25.11.2008
(51) Int. Cl.: B25J 9/16, G05B 19/402, A61B 19/00

(54) **Verfahren zur Ermittlung eines Ankerpunktes eines medizinischen Roboterarms**
Method of determining the anchor point of a medical robot arm
Procédé destiné à détermination du point d'armature d'un bras de robot médical

(43) Veröffentlichungstag der Anmeldung: 26.05.2010
(73) Patentinhaber: BrainLAB AG, 85622 Feldkirchen (DE)
(72) Erfinder: Birkenbach, Rainer, 85435, Erding (DE); Wohlgemuth, Richard, 83646, Bad Tölz (DE)
(74) Vertreter: Schwabe - Sandmair - Marx

(56) Entgegenhaltungen:
- WO-A-99/50721
- WO-A-02/060653
- US-A1- 2006 048 364
- XU ET AL: "An investigation on mobility and stiffness of a 3-DOF translational parallel manipulator via screw theory" ROBOTICS AND COMPUTER INTEGRATED MANUFACTURING, ELSEVIER SCIENCE PUBLISHERS BV., BARKING, GB, Bd. 24, Nr. 3, 1. März 2008 (2008-03-01), Seiten 402-414, XP022504961 ISSN: 0736-5845
- QINGSONG XU ET AL: "Stiffness Optimization of a 3-DOF Parallel Kinematic Machine Using Particle Swarm Optimization" ROBOTICS AND BIOMIMETICS, 2006. ROBIO '06. IEEE INTERNATIONAL CON FERENCE ON, IEEE, PI, 1. Dezember 2006 (2006-12-01), Seiten 1169-1174, XP031068952 ISBN: 978-1-4244-0570-1
- S. MURUGANANDAM & S. PUGAZHENTHI: "Stiffness-based workspace atlas of hexapod machine tool for optimal work piece location" THE INTERNATIONAL JOURNAL OF ADVANCED MANUFACTURING TECHNOLOGY, 17. Juni 2008 (2008-06-17), XP002523699 Springer-Verlag London
- HO S C ET AL: "Establishing a force control strategy incorporating active motion constraint" IEEE ENGINEERING IN MEDICINE AND BIOLOGY MAGAZINE, IEEE SERVICE CENTER, PISACATAWAY, NJ, US, Bd. 14, Nr. 3, 1. Mai 1995 (1995-05-01), Seiten 292-300, XP011084609 ISSN: 0739-5175

## Beschreibung

In medizinischen Anwendungen ist der Einsatz von Roboterarmen bekannt, die beispielsweise ein medizinisches Instrument tragen. Dabei fährt der Roboterarm automatisch eine vorgegebene Trajektorie ab oder wird von einem Bediener geführt. Außerdem ist es möglich, dass der Roboterarm bei einer Berührung oder geringen Krafteinwirkung durch den Benutzer eine vorgegebene Trajektorie abfährt. Das Dokument US 2007/144298 beschreibt beispielsweise einen Roboterarm, dessen Bewegung auf ein vorherbestimmtes Volumen beschränkt ist. Ein Problem bei derartigen Roboterarmen ist eine mögliche Abweichung der Roboterarmposition von der Sollposition.

Es ist die Aufgabe der vorliegenden Erfindung, ein Verfahren bereitzustellen, mit dem sich Ankerpunkt des medizinischen Roboterarms ermitteln lässt.

Diese Aufgabe wird durch die Merkmale der unabhängigen Ansprüche gelöst.

Ein Roboterarm besteht aus zwei oder mehr Armabschnitten, wobei jeweils zwei Armabschnitte über mindestens ein Gelenk miteinander verbunden sind. Der Begriff Gelenk umfasst neben Verbindungselementen, die eine Rotation zwischen zwei Armabschnitten zulassen, auch Verbindungselemente, die eine Translation ermöglichen. Bei einem Armabschnitt handelt es sich beispielsweise um ein Rohr. Als Position des Roboterarms oder Roboterarmposition wird die Position des beweglichen Endes des Roboterarms im Raum bezeichnet. Unter dem Begriff Gelenkstellung wird die Gesamtheit der Stellungen aller Gelenke des Roboterarms verstanden. Je nach Ausgestaltung des Roboterarms ist es möglich, ein und dieselbe Roboterarmposition durch verschiedene Gelenkstellungen zu erreichen. Die Stellung eines Gelenks wird durch ein Stellglied wie zum Beispiel einen Elektromotor erzeugt, wobei optional ein Getriebe vorgesehen ist.

Ferner weist der Roboterarm einen Befestigungspunkt auf, an dem der Roboterarm an einer Struktur wie einem Stativ oder einer sonstigen Halterung befestigt wird. Der Arbeitsraum umfasst eine Menge von Positionen, die der Roboterarm erreichen kann. Dabei kann es sich um ein geschlossenes oder halboffenes Volumen handeln, um eine Fläche oder um eine Linie.

Bei dem Steifigkeitsmodell handelt es sich um ein mathematisches Modell des Roboterarms, mittels dessen sich die Gesamtsteifigkeit des Roboterarms berechnen lässt. Die Steifigkeit beschreibt den Zusammenhang zwischen einer auf den Roboterarm wirkenden Kraft und der Verformung des Roboterarms. Sie ist unter anderem abhängig von den Werkstoffen des Roboterarms und seiner Geometrie. Die Erstellung eines Steifigkeitsmodells ist dem Fachmann bekannt und wird daher nicht näher erläutert.

Die Beurteilung ist ein der Berechnung der Steifigkeit des Roboterarms anhand des Steifigkeitsmodells nachgeordneter Verfahrensschritt, in dem insbesondere die Positionierungsgenauigkeit auf ein Kriterium oder mehrere Kriterien hin untersucht wird. Der Schritt der Beurteilung kann beispielsweise mindestens einen der folgenden Schritte umfassen: die Steifigkeit des Roboterarms in einem abzufahrenden Arbeitsraum mit einer Sollsteifigkeit vergleichen, den Befestigungspunkt des Roboterarms festlegen, eine Hinweisinformation ausgeben, eine optimale aus mehreren möglichen Gelenkstellungen auswählen oder die Roboterarmposition mit einer gemessenen Roboterarmposition vergleichen. Der Schritt der Beurteilung entspricht insbesondere einer Auswertung der Positionierungsgenauigkeit, insbesondere basierend auf der berechneten Steifigkeit.

Aufgrund der begrenzten Steifigkeit des Roboterarms entspricht die tatsächliche Roboterarmposition oftmals nicht exakt der berechneten oder theoretischen Roboterarmposition, wie sie die Gelenkstellung theoretisch vorgibt. Die Abweichung der tatsächlichen von der theoretischen Roboterarmposition, die auch als Positionsabweichung bezeichnet wird, ist ein Maß für die Positionierungsgenauigkeit und kann als Grundlage für weitere Berechnungsschritte dienen.

Die Abweichung berechnet sich aus der Steifigkeit des Roboterarms sowie den auf den Roboterarm wirkenden Kräften. Eine hohe Steifigkeit bedeutet eine geringe Positionsabweichung, also eine hohe Positionierungsgenauigkeit, und umgekehrt. Die Steifigkeit ist unter anderem abhängig von der Gelenkstellung des Roboterarms. Lässt sich eine Roboterarmposition durch verschiedene Gelenkstellungen erreichen, so kann die Steifigkeit für diese Position, also für jede Gelenkstellung, unterschiedlich sein.

In einer Ausgestaltungsform der Erfindung berücksichtigt das Steifigkeztsrnodell die mechanische Steifigkeit. Die mechanische Steifigkeit wird von einem oder mehreren der Faktoren Steifigkeit der Gelenke, Länge der Armabschnitte, Steifigkeit der Armabschnitte und Steifigkeit der Getriebe beeinflusst. Alternativ oder zusätzlich berücksichtigt das Steifigkeitsmodell die Regelsteifigkeit des Roboterarms. Die Regelsteifigkeit hat ihre Ursache in der meist elektronischen Ansteuerung des Roboterarms, die beispielsweise nicht kontinuierlich, sondern zyklisch erfolgt.

Optional wird bei der Beurteilung der Positionierungsgenauigkeit eine auf den Roboterarm einwirkende Kraft berücksichtigt. Eine solche Kraft ist beispielsweise die Kraft auf ein Instrument am beweglichen Ende des Roboterarms oder die Kraft, die ein Bediener auf den Roboterarm ausübt. Die auf den Roboterarm einwirkende Kraft wird bevorzugt mittels Kraftsensoren ermittelt, die beispielsweise im Bereich der Gelenke angeordnet sind. Alternativ kann es sich bei der Kraft um eine hypothetische Kraft handeln, von der angenommen wird, dass sie bei der Benutzung des Roboterarms auf den Roboterarm ausgeübt wird. Dies ist insbesondere bei der Planung des Arbeitsraums vorteilhaft.

Alternativ oder zusätzlich wird bei der Beurteilung der Positionierungsgenauigkeit das Eigengewicht des Roboterarms berücksichtigt. Dazu wird beispielsweise jede berücksichtigte Komponente des Roboterarms, also jedes Gelenk oder jeder Armabschnitt, als punktförmige Masse angenähert und als in diesem Punkt angreifende Gewichtskraft berücksichtigt.

Optional wird die Steifigkeit bzw. die Positionsabweichung nicht in allen Raumrichtungen, sondern nur in ein oder zwei Raumrichtungen berechnet. Dies führt zu einem reduzierten Rechenaufwand und ist insbesondere dann sinnvoll, wenn die Krafteinwirkung nicht aus allen Raumrichtungen erfolgt.

Gemäß einer Ausgestaltungsform der Erfindung wird anhand der Steifigkeit ein vom Roboterann abfahrbarer Arbeitsraum berechnet. Der Arbeitsraum wird durch diejenigen Roboterarmpositionen begrenzt, an denen die Steifigkeit einen Grenzwert erreicht. Eine Roboterarmposition, an der die Steifigkeit unterhalb dieses Grenzwertes läge, ist somit durch den Roboterarm nicht erreichbar. Der Grenzwert wird beispielsweise von einem Bediener festgelegt und/oder auf Basis des Anwendungsfalls des Roboterarms ermittelt. Diese Ausgestaltungsform stellt ein Verfahren zur Überprüfung bzw. zur Beschränkung des Arbeitsraums eines Roboterarms dar. Alternativ wird für einen gegebenen Arbeitsraum eine Steifigkeit, beispielsweise eine minimale, maximale oder durchschnittliche Steifigkeit, berechnet.

Optional wird überprüft, ob der berechnete abfahrbare Arbeitsraum den für eine Anwendung des Roboterarms notwendigen Bereich enthält. Dadurch wird sichergestellt, dass die Steifigkeit des Roboterarms während der gesamten Anwendung einen zulässigen Minimalwert nicht unterschreitet, also bei den auftretenden Kräften eine maximal zulässige Abweichung nicht überschritten wird.

Gemäß der Erfindung wird der Ankerpunkt für den Roboterarm aus der Steifigkeit und dem abzufahrenden Arbeitsraum berechnet. Der Ankerpunkt ist derjenige Punkt im Raum, an dem der Roboterarm mit seinem Befestigungspunkt fixiert ist, beispielsweise an einem Operationstisch. Dabei wird der Ankerpunkt so bestimmt, dass der Roboterarm in dem gesamten abzufahrenden Arbeitsraum eine vorgegebene Steifigkeit einhält, aber nicht unterschreitet. In einer Ausgestaltungsform werden nacheinander mehrere hypothetische Ankerpunkte angenommen und daraus derjenige Ankerpunkt ausgewählt, für den die Steifigkeit in dem überprüften Arbeitsraum den größten Minimalwert oder den größten Mittelwert aufweist. Diese Anwendung stellt ein Verfahren zur Ermittlung des Ankerpunktes eines Roboterarms dar.

Optional werden Anweisungen an den Benutzer gegeben, wie der Roboterarm relativ zu einer (anatomischen) Struktur zu positionieren ist, so dass der für die Anwendung erforderliche (abzufahrende) Arbeitsraum innerhalb des mit der gewünschten Steifigkeit erreichbaren Arbeitsraums liegt. Bei der Berechnung des Ankerpunktes können insbesondere die Navigationsdaten eingehen, die die Lage der (anatomischen) Struktur beschreiben. Über die Navigationsdaten ergibt sich auch die Lage des abzufahrenden Arbeitsraums im Raum.

Optional kann eine Warnung ausgegeben werden, wenn der Roboterarm eine Position erreicht, an der ein festgelegter Grenzwert für die Steifigkeit unterschritten wird. Dies ist insbesondere für den Anwendungsfall sinnvoll, in dem der Bediener den Roboterarm mit nur geringer Kraftausübung über eine vorherbestimmte Trajektorie schiebt oder der Arbeitsraum nicht beschränkt ist. Diese Anwendung stellt ein Verfahren zur Kontrolle des Arbeitsraums bei der aktuell einwirkenden Kraft eines Roboterarms dar. Weiterhin optional wird ein Zahlenwert der Positionierabweichung und/oder der Steifigkeit für die aktuelle Gelenkstellung angezeigt oder angegeben.

Es ist möglich, eine Warnung auszugeben, wenn die von dem Benutzer und/oder dem Instrument auf den Roboterarm ausgeübte Kraft einen Grenzwert überschreitet. Zwischen diesen beiden Kräften kann beispielsweise über deren Angriffspunkte unterschieden werden. Dazu sind beispielsweise mindestens zwei Kraftsensoren vorgesehen.

Bevorzugt wird zur Einnahme einer Roboterarmposition diejenige von mehreren möglichen Gelenkstellungen ausgewählt, für die festgelegte Kriterien an die Steifigkeit erfüllt werden. Ein Kriterium kann beispielsweise eine minimale Steifigkeit sein. Wird eine Gelenkstellung gefunden, die eine festgelegte Steifigkeit erreicht oder überschreitet, so wird diese Gelenkstellung ausgewählt. Alternativ kann diejenige Gelenkstellung ausgewählt werden, bei der die größte Steifigkeit erreicht wird. Zur Überprüfung der Steifigkeit auf das Kriterium oder die Kriterien wird die Steifigkeit anhand des Steifigkeitsmodells für verschiedene Gelenkstellungen berechnet und beurteilt. Diese Anwendung stellt ein Verfahren zur Optimierung der Gelenkstellung eines Roboterarms dar.

Soll der Roboterarm während einer Anwendung verschiedene Positionen einnehmen, so ist es in den meisten Fällen unerwünscht, bei benachbarten Roboterarmpositionen stark unterschiedliche Gelenkstellungen vorzufinden. In diesem Fall würde der Roboterarm bei nur einer geringen Positionsänderung eine unstetige Änderung der Gelenkstellung ausführen, was eine große Bewegung des Roboterarms, insbesondere der Gelenke oder der Armabschnitte, bedeutet. In diesem Fall ist es wünschenswert, bestimmten Roboterarmpositionen Gelenkstellungen zuzuweisen, die nicht notwendigerweise die maximale Steifigkeit erreichen, jedoch eine stetige Änderung der Gelenkstellungen bei einer Veränderung der Roboterarmposition ermöglichen. Stetigkeit liegt beispielsweise dann vor, wenn zwischen einer ersten Gelenkstellung für eine erste Roboterarmposition und einer zweiten Gelenkstellung für eine zweite Roboterarmposition die Änderung der Position jedes Gelenks im Raum geringer ist als der Abstand der beiden Roboterarmpositionen, insbesondere kleiner ist als 1 mal, 0,9 mal, 0,8 mal, 0,75 mal, 0,5 mal, 0,25 mal oder 0,1 mal der Abstand der beiden Roboterarmpositionen. Dies gilt für den Endzustand nach der Neupositionierung und/oder den Übergang von einer Roboterarmposition in die andere.

Es wird bevorzugt eine Gelenkstellung ausgewählt, von der aus der gesamte Arbeitsraum durch ausschließlich stetige Änderungen der Gelenkstellung erreichbar ist, ohne eine minimal geforderte Steifigkeit zu unterschreiten. Gibt es mehrere solcher Gelenkstellungen, so wird beispielsweise diejenige ausgewählt, für die bei der momentanen Roboterarmposition die größte Steifigkeit vorliegt, die am wenigsten von der momentanen Gelenkstellung abweicht oder für die ein Parameter maximiert wird. Ein solcher Parameter ist insbesondere die minimale, maximale oder mittlere Steifigkeit des Roboterarms im Arbeitsraum. Für die Auswahl der Gelenkstellung wird die Steifigkeit anhand des Steifigkeitsmodells für verschiedene Gelenkstellungen und Roboterarmpositionen berechnet und beurteilt.

Erfindungsgemäß wird bei der Berechnung des Ankerpunktes des Roboterarms die Steifigkeit optimal über den abzufahrenden Arbeitsraum gemittelt. Bei der Mittelwertbildung werden, Positionen in unterschiedlichen Bereichen des Arbeitsraums unterschiedliche Gewichtungsfaktoren angeordnet, um in wichtigen Bereichen des Arbeitsraums eine größere Steifigkeit sicherzustellen.

Optional erfolgt ein Vergleich der anhand des Steifigkeitsmodells berechneten Roboterarmposition mit einer gemessenen Roboterarmposition. Dazu ist das bewegliche Ende des Roboterarms bevorzugt mit einer Markervorrichtung oder einem Referenzstern versehen. Aus der Differenz der beiden Positionen und den auf den Roboterarm wirkenden Kräften ist es möglich, das Steifigkeitsmodell zu aktualisieren. Außerdem kann eine Warnung ausgegeben werden, wenn die Differenz der Positionen einen Grenzwert überschreitet. Alternativ kann die auf den Roboterarm einwirkende Kraft aus der gemessenen Position und der Steifigkeit des Roboterarms berechnet werden.

Unter einer Markervorrichtung können ein einzelner, aber auch mehrere Marker verstanden werden. Aufgabe eines Markers ist, von einer Markererfassungsrichtung (z.B. einer Kamera) erfasst zu werden, so dass seine Lage (d.h. Position und/oder Ausrichtung) im Raum ermittelt werden kann. Solche Marker können aktive Marker sein. Ein aktiver Marker emittiert beispielsweise elektromagnetische Strahlung bzw. Wellen, wobei diese Strahlung im infraroten, sichtbaren und/oder ultravioletten Spektralbereich liegen kann. Der Marker kann aber auch passiv sein, d.h. beispielsweise elektromagnetische Strahlung aus dem infraroten, sichtbaren und/oder ultravioletten Spektralbereich reflektieren. Dazu kann der Marker mit einer Oberfläche versehen sein, die entsprechende Reflektionseigenschaften besitzt. Es ist auch möglich, dass ein Marker elektromagnetische Strahlung bzw. Wellen reflektiert und/oder emittiert, die im Bereich der Radiofrequenzen oder bei Ultraschallwellenlängen liegt. Ein Marker besitzt vorzugsweise sphärische Form bzw. eine kugelähnliche Gestalt und kann daher als Markerkugel bezeichnet werden.

Ein Referenzstern bezeichnet eine Einrichtung, an der mehrere, vorteilhaft drei Markervorrichtungen angebracht sind. Die Markervorrichtungen sind dabei ortsfest und vorteilhaft lösbar an dem Referenzstern angebracht, so dass eine bekannte relative Lage der Markervorrichtungen zueinander entsteht. Der Referenzstern dient dazu, in einem chirurgischen Navigationsverfahren eine Mehrzahl von Markervorrichtungen an einem Gegenstand (beispielsweise einem Knochen oder einem medizinischen Instrument) anzubringen, um die Lage des Gegenstandes im Raum (d.h. seine Position und/oder Ausrichtung) erfassen zu können. Ein solcher Referenzstern umfasst für gewöhnlich eine Anbringmöglichkeit an dem Gegenstand (beispielsweise eine Schelle und/oder ein Gewinde), ein Halteelement, das für einen Abstand der Markervorrichtungen von dem Gegenstand sorgt (um insbesondere die Sichtbarkeit der Markervorrichtungen für eine Markererfassungseinrichtung zu unterstützen) sowie mit dem Halteelement mechanische verbundene Markerhalter, an die die Markervorrichtungen angebracht werden können. Wo es aus dem Zusammenhang klar wird, kann der Begriff Referenzstern auch einen Referenzstern mit wenigstens einer daran angebrachten Markervorrichtung bezeichnen. Ein solches System aus einem Referenzstern und wenigstens einer Markervorrichtung kann auch Markerstern genannt werden.

Optional wird die Steifigkeit an der aktuellen Roboterarmposition vom Benutzer als neue Grenzsteifigkeit ausgewählt, wobei der Arbeitsraum aufgrund dieser neuen Grenzsteifigkeit erneut berechnet wird.

Weiterhin optional erfolgt eine automatische Korrektur der Gelenkstellung. Dabei werden die Gelenke so angesteuert, dass die aus der Steifigkeit und den auf den Roboterarm einwirkenden Kräften berechnete Positionsabweichung kompensiert wird.

Es ist möglich, verschiedene Merkmale oder Anwendungen der vorliegenden Erfindung miteinander zu kombinieren. Anstatt der Steifigkeit kann jeweils auch die Positionsabweichung als Kriterium verwendet werden, die sich wie bereits beschrieben aus der Steifigkeit und den auf den Roboterarm wirkenden Kräften berechnet. Dabei ist die Vergleichslogik anzupassen. Soll die Steifigkeit zum Beispiel einen Grenzwert nicht unterschreiten, so darf die Positionsabweichung einen Grenzwert nicht überschreiten. Eine Maximierung der Steifigkeit bedeutet eine Minimierung der Positionsabweichung, und so weiter.

Die vorliegende Erfindung betrifft weiterhin ein Softwareprogramm, das dazu ausgebildet ist, das vorstehende Verfahren auszuführen. Dazu wird das Softwareprogramm beispielsweise auf einer Recheneinheit ausgeführt, die mit einem geeigneten Roboterarm verbunden ist.

Die vorliegende Erfindung betrifft weiterhin eine Steuereinrichtung für einen medizinischen Roboterarm mit mindestens einem Gelenk, die dazu eingerichtet ist, das vorstehend beschriebene Verfahren auszuführen, sowie ein Operationssystem mit einer solchen Steuereinrichtung und einem Roboterarm.

Die Erfindung soll anhand eines Ausführungsbeispiels näher erläutert werden. Dabei zeigt:

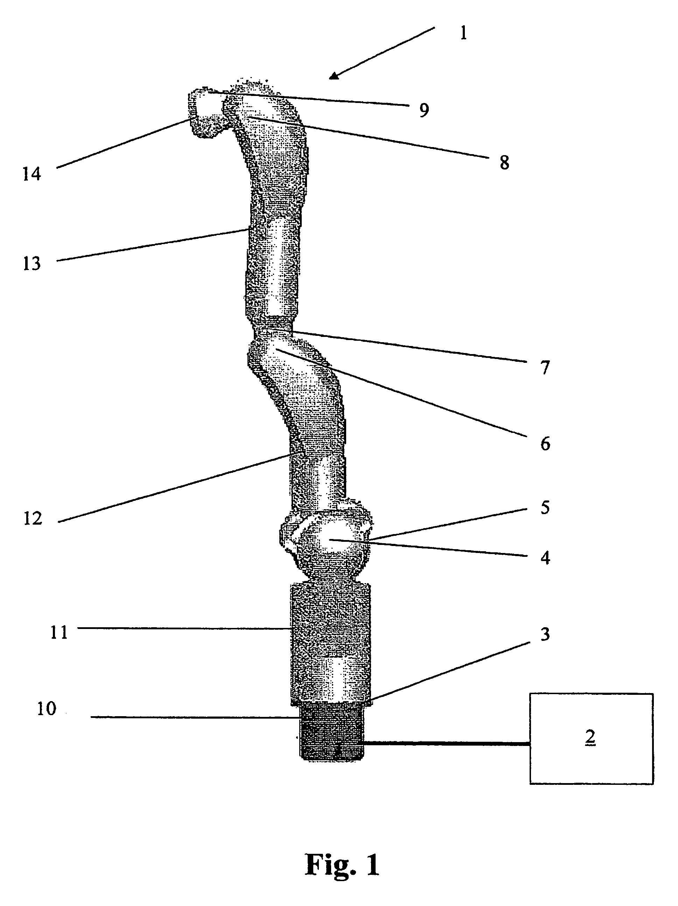

- Figur 1: einen medizinischen Roboterarm mit sieben Gelenken.

Die Figur 1 zeigt einen Roboterarm 1 mit sieben Gelenken 3-9. Der Roboterarm 1 ist mit einer Steuereinheit 2 verbunden, die nicht dargestellte Antriebe zur Verstellung der Gelenke 3-9 im Roboterarm 1 ansteuert. Jeder Antrieb besteht beispielsweise aus einem Elektromotor, der die Stellung des zugehörigen Gelenks verändert, wobei optional ein Getriebe vorgesehen ist. Der Roboterarm besteht weiterhin aus vier Armabschnitten 11, 12, 13, 14 sowie einem Basisteil 10. Das Basisteil 10 dient der Befestigung des Roboterarms 1, der Befestigungspunkt des Roboterarms 1 befindet sich also am Basisteil 10. Der Armabschnitt 14 am beweglichen Ende des Roboterarms 1 bildet gleichzeitig eine Haltevorrichtung 14 für ein medizinisches Instrument. Die Position der Haltevorrichtung 14 im Raum kann auch als Roboterarmposition bezeichnet werden.

Der Armabschnitt 11 ist über das Gelenk 3 in einer Achse gegenüber dem Trägerteil 10 verdrehbar. Der Armabschnitt 12 ist über die Gelenke 4,5 in zwei Achsen gegenüber dem Armabschnitt 11 verdrehbar. Der Armabschnitt 13 ist über die Gelenke 6, 7 um zwei Achsen gegenüber dem Armabschnitt 12 verdrehbar. Die Aufnahmevorrichtung 14 ist über die Gelenke 8,9 um zwei Achsen gegenüber dem Armabschnitt 13 verdrehbar. Die Gelenke 4, 5, 6, 8 ermöglichen eine Schwenkbewegung, die Gelenke 3,7,9 ermöglichen eine Torsionsbewegung.

Die Haltevorrichtung 14 dient der Aufnahme eines medizinischen Instruments wie beispielsweise einer Biopsienadel oder einem Fräskopf. Der Roboterarm 1 dient dazu, das medizinische Instrument präzise zu positionieren und zu verfahren.

Unter anderem aufgrund der Materialsteifigkeit der Armabschnitte 11-14 sowie der Gelenke 3-9 kommt es zu einer Abweichung der Roboterarmposition, und damit der Position des medizinischen Instruments, von der theoretischen Position, die sich aus der Ansteuerung der Gelenke 3-9 ergibt. Dies wird einerseits durch das Eigengewicht des Roboterarms 1 und andererseits durch externe, auf den Roboterarm 1 einwirkende Kräfte verursacht. Die einwirkenden Kräfte werden von nicht dargestellten Kraftsensoren ermittelt, die beispielsweise in die Gelenke 3-9 integriert sind.

Zur Beurteilung der Positionsabweichungen ist in der Steuereinheit 2 ein Steifigkeitsmodell des Roboterarms 1 hinterlegt. Anhand des Steifigkeitsmodells und der wirkenden Kräfte lässt sich die Abweichung der tatsächlichen Roboterarmposition von der theoretischen Roboterarmposition in Abhängigkeit von der Gelenkstellung berechnen. Das Ergebnis dieser Berechnung bzw. die Steifigkeit alleine kann auf vielfältige Art und Weise verwendet werden.

Eine erste Möglichkeit besteht darin, eine minimale Steifigkeit vorzugeben und daraus den Arbeitsraum zu berechnen, innerhalb dessen der Roboterarm 1 die geforderte Steifigkeit einhält. Bei der anschließenden Verwendung des Roboterarms 1 wird eine Roboterarmposition außerhalb dieses Arbeitsraums von der Steuereinheit 2 unterbunden. Insbesondere kann vor der Verwendung des Roboterarms 1 überprüft werden, ob der mit der gewünschten Steifigkeit abgedeckte Arbeitsraum den für die Anwendung notwendigen Raum enthält. Die gewünschte minimale Steifigkeit wird beispielsweise vom Bediener des Roboterarms 1 eingegeben oder automatisch anhand des Anwendungsfalls gewählt.

Gemäß der Erfindung wird der Ankerpunkt des Roboterarms 1, also die Position des Basisteils 10, aus der Steifigkeit und dem abzufahrenden Arbeitsraum berechnet. Dabei werden beispielsweise iterativ verschiedene Ankerpunkte angenommen und zu jedem Ankerpunkt der Arbeitsraum berechnet, der von dem Roboterarm 1 mit der gewünschten Steifigkeit abgedeckt wird.

Beispielsweise wird der erste Ankerpunkt gewählt, bei dem die Steifigkeit für einen gesamten zu erreichenden Arbeitsraum eingehalten wird. Alternativ wird für jeden Ankerpunkt und den zu erreichenden Arbeitsraum die minimale Steifigkeit berechnet und der Ankerpunkt mit der größten minimalen Steifigkeit gewählt. Die minimale Steifigkeit ist die Steifigkeit des Roboterarms 1 in derjenigen Position innerhalb des gesamten Arbeitsraums, für die die Steifigkeit des Arms 1 am geringsten ist. Weiter alternativ wird der Ankerpunkt mit der größten mittleren Steifigkeit gewählt, für den die Anforderungen an die minimale Steifigkeit erfüllt werden. Die mittlere Steifigkeit ist beispielsweise das arithmetische Mittel aller Steifigkeiten. Dabei werden die Steifigkeiten entweder gleich gewichtet oder für verschiedene Bereiche des abzufahrenden Arbeitsraums unterschiedlich gewichtet.

Nach einer dritten Möglichkeit wird eine Warnung ausgegeben, wenn der Roboterarm 1 einen festgelegten Grenzwert für die Steifigkeit unterschreitet. Bewegt der Bediener den Roboterarm 1 in eine Position, an der der Grenzwert für die Steifigkeit unterschritten wird, so ertönt beispielsweise ein Warnton oder es wird ein Warnsignal angezeigt.

Gemäß einer vierten Möglichkeit wird zur Erreichung einer Roboterarmposition diejenige von mehreren möglichen Gelenkstellungen ausgewählt, für die festgelegte Kriterien an die Steifigkeit erfüllt werden. Dies ist möglich, wenn die Anzahl der Gelenke überbestimmt ist, eine Roboterarmposition also durch mehrere Gelenkstellungen erreicht werden kann. In diesem Fall ist die optimale Gelenkstellung zu bestimmen.

Dazu wird beispielsweise die Menge aller möglichen Gelenkstellungen bestimmt und nacheinander für jede Gelenkstellung überprüft, ob sie zu der gewünschten minimalen Steifigkeit führt. Es wird dann beispielsweise die erste Gelenkstellung ausgewählt, die die Steifigkeit erfüllt. Alternativ wird die Berechnung der Steifigkeit für jede mögliche Gelenkstellung durchgeführt und diejenige Gelenkstellung ausgewählt, die die größte minimale oder mittlere Steifigkeit erzielt.

Es ist im Rahmen der vorliegenden Erfindung möglich, einzelne Merkmale der vorgenannten Möglichkeiten zu kombinieren. Insbesondere ist es möglich, beispielsweise bei der Bestimmung des Ankerpunkts die Steifigkeit jeweils für verschiedene Gelenkstellungen zu berechnen.

## Patentansprüche

1. Verfahren zur Ermittlung eines Ankerpunktes eines medizinischen Roboterarms (1) mit mindestens einem Gelenk (3-9), wobei die Steifigkeit des Roboterarms (1) anhand eines Steifigkeitsmodells berechnet und beurteilt wird, **dadurch gekennzeichnet, dass** der Ankerpunkt des Roboterarms (1) aus der Steifigkeit und dem abzufahrenden Arbeitsraum berechnet wird, indem mehrere hypothetische Ankerpunkte angenommen werden und daraus derjenige Ankerpunkt ausgewählt wird, für den die Steifigkeit in dem abzufahrenden, also für eine Anwendung erforderlichen, Arbeitsraum den größten Mittelwert aufweist, wobei bei der Mittelwertbildung Positionen in unterschiedlichen Bereichen des abzufahrenden Arbeitsraums unterschiedliche Gewichtungsfaktoren zugeordnet sind.

2. Verfahren nach Anspruch 1, **dadurch gekennzeichnet, dass** das Steifigkeitsmodell die mechanische Steifigkeit, insbesondere die Steifigkeit der Gelenke (3-8) und/oder der Armabschnitte (11-14), berücksichtigt.

3. Verfahren nach einem der Ansprüche 1 oder 2, **dadurch gekennzeichnet, dass** das Steifigkeitsmodell die Regelsteifigkeit berücksichtigt.

4. Verfahren nach einem der Ansprüche 1 bis 3, **dadurch gekennzeichnet, dass** eine auf den Roboterarm (1) einwirkende Kraft berücksichtigt wird.

5. Verfahren nach einem der Ansprüche 1 bis 4, **dadurch gekennzeichnet, dass** das Eigengewicht des Roboterarms (1) berücksichtigt wird.

6. Verfahren nach einem der Ansprüche 1 bis 5, **dadurch gekennzeichnet, dass** anhand der Steifigkeit ein vom Roboterarm (1) abfahrbarer Arbeitsraum berechnet wird oder für einen gegebenen Arbeitsraum eine Steifigkeit berechnet wird.

7. Verfahren nach Anspruch 6, **gekennzeichnet durch** eine Überprüfung, ob der berechnete Arbeitsraum den für eine Anwendung des Roboterarms (1) notwendigen Bereich enthält.

8. Verfahren nach einem der Ansprüche 1 bis 7, **dadurch gekennzeichnet, dass** eine Warnung ausgegeben wird, wenn die Steifigkeit des Roboterarms (1) einen festgelegten Grenzwert unterschreitet.

9. Verfahren nach einem der Ansprüche 1 bis 8, **gekennzeichnet durch** einen Vergleich einer anhand des Steifigkeitsmodells berechneten Roboterarmposition mit einer gemessenen Roboterarmposition.

10. Softwareprogramm, das, wenn es auf einem Computer läuft oder in einem Computer geladen ist, den Computer dazu veranlasst, das Verfahren nach einem der Ansprüche 1 bis 9 auszuführen.

11. Steuereinrichtung (2) für einen medizinischen Roboterarm (1) mit mindestens einem Gelenk (3-9), die dazu eingerichtet ist, das Verfahren nach einem der Ansprüche 1 bis 9 auszuführen.

12. Operationssystem, aufweisend einen Roboterarm (1) und eine Steuereinrichtung (2) nach Anspruch 11.

## Claims

1. A method for ascertaining an anchoring point of a medical robot arm (1) comprising at least one joint (3-9), wherein the rigidity of the robot arm (1) is calculated and assessed on the basis of a rigidity model, **characterised in that** the anchoring point of the robot arm (1) is calculated from the rigidity and the working space to be travelled, by assuming a number of hypothetical anchoring points and selecting from them the anchoring point for which the rigidity exhibits the greatest average value within the working space to be travelled, i.e. the working space required for an application, wherein when averaging, different weighting factors are assigned to positions in different regions of the working space to be travelled.

2. The method according to claim 1, **characterised in that** the rigidity model takes into account the mechanical rigidity, in particular the rigidity of the joints (3-9) and/or arm sections (11-14).

3. The method according to any one of claims 1 or 2, **characterised in that** the rigidity model takes into account the control rigidity.

4. The method according to any one of claims 1 to 3, **characterised in that** a force acting on the robot arm (1) is taken into account.

5. The method according to any one of claims 1 to 4, **characterised in that** the inherent weight of the robot arm (1) is taken into account.

6. The method according to any one of claims 1 to 5, **characterised in that** a working space which can be travelled by the robot arm (1) is calculated on the basis of the rigidity, or a rigidity is calculated for a given working space.

7. The method according to claim 6, **characterised by** a check as to whether the calculated working space contains the region necessary for an application of the robot arm (1).

8. The method according to any one of claims 1 to 7, **characterised in that** a warning is outputted when the rigidity of the robot arm (1) falls below a defined boundary value.

9. The method according to any one of claims 1 to 8, **characterised by** a comparison between a robot arm position calculated on the basis of the rigidity model and a measured robot arm position.

10. A software program which, when it is running on a computer or is loaded onto a computer, causes the computer to perform the method according to any one of claims 1 to 9.

11. A control device (2) for a medical robot arm (1) comprising at least one joint (3-9), which is configured to perform the method according to any one of claims 1 to 9.

12. An operation system, comprising a robot arm (1) and a control device (2) according to claim 11.

## Revendications

1. Procédé pour déterminer un point d'ancrage d'un bras de robot médical (1) comportant au moins une articulation (3-9), dans lequel la rigidité du bras de robot (1) est calculée et évaluée au moyen d'un modèle de rigidité, **caractérisé en ce que** le point d'ancrage du bras de robot (1) est calculé à partir de la rigidité et de l'espace de travail à parcourir, dans lequel plusieurs points d'ancrage hypothétiques sont supposés et chaque point d'ancrage est choisi à partir de ceux pour lesquels la rigidité dans l'espace de travail à parcourir, c'est-à-dire celui nécessaire pour une application, a la plus grande valeur moyenne, dans lequel lors de la formation de la valeur moyenne, différents facteurs de poids sont attribués à des positions dans différentes zones de l'espace de travail à parcourir.

2. Procédé selon la revendication 1, **caractérisé en ce que** le modèle de rigidité tient compte de la rigidité mécanique, en particulier de la rigidité des articulations (3-8) et/ou des parties de bras (11-14).

3. Procédé selon l'une des revendications 1 ou 2, **caractérisé en ce que** le modèle de rigidité tient compte de la rigidité de régulation.

4. Procédé selon l'une des revendications 1 à 3, **caractérisé en ce qu'**une force agissant sur le bras de robot (1) est prise en compte.

5. Procédé selon l'une des revendications 1 à 4, **caractérisé en ce que** le poids propre du bras de robot (1) est pris en compte.

6. Procédé selon l'une des revendications 1 à 5, **caractérisé en ce qu'**un espace de travail pouvant être parcouru par le bras de robot (1) est calculé au moyen de la rigidité, ou bien une rigidité est calculée pour un espace de travail donné.

7. Procédé selon la revendication 6, **caractérisé par** une vérification pour déterminer si l'espace de travail calculé contient la région nécessaire pour une application du bras de robot (1).

8. Procédé selon l'une des revendications 1 à 7, **caractérisé en ce qu'**un avertissement est généré lorsque la rigidité du bras de robot (1) franchit une valeur limite définie.

9. Procédé selon l'une des revendications 1 à 8, **caractérisé par** une comparaison d'une position de bras de robot calculée au moyen du modèle de rigidité avec une position de bras de robot mesurée.

10. Programme logiciel qui, lorsqu'il s'exécute sur un ordinateur ou lorsqu'il est chargé dans un ordinateur, amène l'ordinateur à mettre en oeuvre le procédé selon l'une des revendications 1 à 9.

11. Dispositif de commande (2) pour un bras de robot médical (1) comportant au moins une articulation (3-9), qui est conçu pour mettre en oeuvre le procédé selon l'une des revendications 1 à 9.

12. Système d'opération comportant un bras de robot (1) et un dispositif de commande (2) selon la revendication 11.
